# EUROPEAN PATENT APPLICATION

(11) **EP 2 476 748 A1**
(43) Date of publication of application: **18.07.2012**
(21) Application number: 10815420.4
(22) Date of filing: 09.09.2010
(51) Int. Cl.: C12N 5/02, C12N 15/09, C12N 5/10

(54) **CULTURE METHOD FOR HEMATOPOIETIC STEM CELLS**

(30) Priority: 10.09.2009 JP 2009209763
(71) Applicant: Keio University, Tokyo 108-8345 (JP)
(72) Inventor: NAKAJIMA, Hideaki, Tokyo 160-8582 (JP)
(74) Representative: Rees, Kerry
(86) International application number: PCT/JP2010/065528
(87) International publication number: WO 2011/030825

(57) **Abstract**

By culturing hematopoietic stem cells in the presence of SFRP-F protein, hematopoietic stem cells for hematopoietic stem cell transplantation can be produced.

SEQUENCE LISTING

<110>  Keio University

<120>  Hematopoietic stem cell culture method

<130>  HMJ05037EP

<140>  EP10815420.4
<141>  2010-9-09

<150>  JP2009-209763
<151>  2009-9-10

<160>  4

<170>  PatentIn version 3.5

<210>  1
<211>  26
<212>  DNA
<213>  Artificial Sequence

<220>
<223>  PCR primer

<400>  1
ggatccatgg gcgtcgggcg cagcgc                                          26


<210>  2
<211>  26
<212>  DNA
<213>  Artificial Sequence

<220>
<223>  PCR primer

<400>  2
gaattctcac ttaaaaacag actgga                                          26


<210>  3
<211>  26
<212>  DNA
<213>  Artificial Sequence

<220>
<223>  PCR primer

<400>  3
ggatccatgc cgcggggccc tgcctc                                          26


<210>  4
<211>  26
<212>  DNA
<213>  Artificial Sequence

<220>
<223>  PCR primer

<400>  4
gaattcctag cattgcagct tgcgga                                          26

## Description

### CROSS REFERENCE TO RELATED APPLICATIONS

This application claims the benefit of priority to Japanese Patent Application No. 2009-209763, filed on September 10, 2009, which is incorporated herein by reference.

### TECHNICAL FIELD

The present invention relates to methods for culturing hematopoietic stem cells.

### BACKGROUND ART

Hematopoietic stem cells refer to stem cells that can differentiate into all blood lineage cells, such as leucocytes (including neutrophils, eosinophils, basophils, and lymphocytes), erythrocytes, platelets, etc. Hematopoietic stem cells reside mainly in the bone marrow, which is a hematopoietic organ, in human adults, and sustain life-long production of blood cells by having two functions: self-renewal and differentiation into each type of blood cells. Hematopoietic stem cells also reside in umbilical cord blood, and are present in peripheral blood during leukocyte recovery after chemotherapy and in the administration of G-CSF.

A therapeutic strategy in which hematopoietic stem cells are transplanted in a patient with decreased hematopoietic ability to recover the hematopoietic ability is referred to as hematopoietic stem cell transplantation. Hematopoietic stem cell transplantation has two main goals. One goal is to recover the hematopoietic function in patients with diseases accompanied by decreased hematopoietic function, such as aplastic anemia, by taking advantage of hematopoietic stem cell transplantation. The other goal is to recover hematopoietic cells which have been damaged by pre-treatment with high-dose anticancer drug or radiation, which kills tumor cells, in patients with hematologic malignancies, such as leukemia, myelodysplastic syndromes, malignant lymphoma, and multiple myeloma, or certain type of solid cancer.

As described above, since hematopoietic stem cells reside in the bone marrow, umbilical cord blood, and peripheral blood, peripheral blood-isolated hematopoietic stem cells, bone marrow, and umbilical cord blood are used as transplants for hematopoietic stem cell transplantation. They have their own advantages and disadvantages, and are properly used depending on the purpose of transplantation. In transplantations using these hematopoietic stem cells, it is expected that better treatment results will be achieved by efficiently proliferating hematopoietic stem cells, or by improving integration rate of transplanted hematopoietic stem cells and thus improving their hematopoietic ability and long-term bone marrow-repopulating ability. By way of example, a method has been developed for improving the physiological properties of hematopoietic stem cells by adding TIMP-3 to the culture medium of hematopoietic stem cells (W02007/148609).

### DISCLOSURE OF THE INVENTION

### PROBLEMS TO BE SOLVED BY THE INVENTION

The present invention has been made to provide methods for culturing hematopoietic stem cells, by which hematopoietic stem cells for hematopoietic stem cell transplantation can be produced.

### MEANS FOR SOLVING THE PROBLEM

The method for culturing hematopoietic stem cells includes the step of culturing the hematopoietic stem cells in the presence of a SFRP-2. For that purpose, the SFRP-2 protein may be added to the medium, or an expression vector of the SFRP-2 protein may be introduced into the hematopoietic stem cells. In any one of the aforementioned culture methods, the concentration of the SFRP-2 protein in the medium may be in the range of 0.1 µg/mL or more to 10 µg/mL or less.

Any of the above-mentioned culture methods may include the step of isolating CD-34-negative, Sca-1-positive, c-Kit-positive, and lineage-antigen-negative hematopoietic stem cells from bone marrow cells or blood cells. The aforementioned lineage antigen may be a set of antigens consisting of CD5, CD45R (B220), CD11b, Gr-1 (Ly-6G/C), 7-4, and Ter-119.7.

The agent for improving a function of hematopoietic stem cells according to the present invention contains a SFRP-2 protein or an expression vector that expresses the SFRP-2 protein.

The kit for culturing hematopoietic stem cells according to the present invention includes a SFRP-2 protein or an expression vector that expresses the SFRP-2 protein.

### BRIEF DESCRIPTION OF THE DRAWINGS

FIG. 1 is a graph showing the results obtained by examining in vitro proliferation of hematopoietic stem cells in one example of the present invention.
FIG.2 is a graph showing the results obtained from the colony assay using hematopoietic stem cells in one example of the present invention (*: p<0.05).
FIG. 3 is a graph showing the results obtained by examining the percentage of donor-derived cells in the peripheral blood of the mice into which CD34-KSL cells have been transplanted in one example of the present invention (*: p<0.05).

### BEST MODE FOR CARRYING OUT THE INVENTION

Embodiments of the present invention accomplished based on the above-described findings are hereinafter described in detail by giving Examples. Unless otherwise explained, methods described in standard sets of protocols such as J. Sambrook and E. F. Fritsch & T. Maniatis (Ed.), "Molecular Cloning, a Laboratory Manual (3rd edition), Cold Spring Harbor Press and Cold Spring Harbor, New York (1989) ; and F. M. Ausubel, R. Brent, R. E. Kingston, D. D. Moore, J. G. Seidman, J. A. Smith, and K. Struhl (Ed.),"Current Protocols in Molecular Biology," John Wiley & Sons Ltd., and/or their modified/changed methods are used. When using commercial reagent kits and measuring apparatus, unless otherwise explained, protocols attached to them are used.

The object, characteristics, and advantages of the present invention as well as the idea thereof will be apparent to those skilled in the art from the descriptions given herein. It is to be understood that the embodiments and specific examples of the invention described herein below are to be taken as preferred examples of the present invention. These descriptions are only for illustrative and explanatory purposes and are not intended to limit the invention to these embodiments or examples. It is further apparent to those skilled in the art that various changes and modifications may be made based on the descriptions given herein within the intent and scope of the present invention disclosed herein.

### == Culture of hematopoietic stem cells ==

First, hematopoietic stem cells to be used in the culture method according to the present invention are prepared. The animal species from which hematopoietic stem cells are derived are not particularly limited, but preferred are vertebrates, more preferred are mammals, and the most preferred and humans. The tissues from which hematopoietic stem cells are obtained are not particularly limited as long as the tissue contains hematopoietic stem cells, but preferred are hematopoietic tissues. In human adults, preferred is the bone marrow, umbilical cord blood, or peripheral blood. When the tissues obtained are cell aggregates, they may be dissociated by treatment with protease, collagenase, or the like, into separated discrete cells, which may be used for culture without any further treatment. Tissues such as blood, in which cells are discrete, may be used as they are for culture without dissociation treatment of cells. Alternatively, by isolating hematopoietic stem cells from the dissociated cells, only the hematopoietic stem cells may be used.

Markers for isolating hematopoietic stem cells may be selected by a conventional way of those skilled in the art. In mice, for example, using CD34-negative, Sca-1- positive, c-Kit-positive, and lineage antigens-negative property, cell populations containing hematopoietic stem cells with long-term bone marrow-repopulating ability at a high proportion can be obtained. Using CD34- positive, Sca-1-positive, c-Kit-positive, and lineage antigens-negative property, cell populations containing hematopoietic stem cells with short-term bone marrow-repopulating ability at a high proportion can be obtained. In humans, using CD34-positive, CD38-negative, and lineage antigen-negative property, cell populations containing hematopoietic stem cells at a high proportion can be obtained. Within the resulting cell populations, the proportion of hematopoietic stem cells may be low, but high proportion is preferred. As used herein, lineage antigens refer to marker antigens of blood cells other than hematopoietic stem cells, exemplified by a set of antigens: CD5, CD45R (B220), CD11b, Gr-1 (Ly-6 G/C), 7-4, and Ter-119. The isolation method is not particularly limited, either; cells possessing each marker property can be isolated using the antibodies, each of which has been made against each of the markers, by FACS, magnetic beads, or the like.

The culture medium for hematopoietic stem cells may be selected according to a conventional way known to those skilled in the art. Exemplary culture media include, without limitation, α-MEM+10% FBS, commercial serum-free media for hematopoietic stem cell culture, such as S-Clone SF-02 (Sanko Junyaku Co., Ltd.) and StemPro-34 (Invitrogen).

In the culture method according to the present invention, hematopoietic stem cells are cultured in the presence of SFRP-2 protein. The specific method for culture in this manner is not particularly limited. SFRP-2 protein may be exogenously added to the medium. SFRP-2 expression vector may be introduced into cultured cells, and their supernatant containing expressed SFRP-2 protein may be added; or alternatively, SFRP-2 expression vector may be introduced into hematopoietic stem cells. The method for preparing SFRP-2 protein to be added is also not particularly limited, and SFRP-2 protein may be purified from cells expressing the protein; or alternatively, may be produced as a recombinant protein using E. coli, cultured cells, or the like. The degree of purification of SFRP-2 protein is not particularly limited, either.

The origin of the SFRP-2 protein is not particularly limited. The protein may be derived from either human SFRP-2 (mRNA: Protein: NM_003013, NP_003004) or mouse SFRP-2 (mRNA: Protein: NM_009144, NP_033170); or alternatively, homologues or orthologues of other animals, but it is preferred that the species of the homologues or orthologues is the same as that of the animal from which the hematopoietic stem cells are derived. The concentration of SFRP-2 protein in the medium is not particularly limited, but a preferred range is 0.1 µg/mL or more to 10 µg/mL or more, a more preferred range is 0.5 µg/mL or more to 5 µg/mL or more, and the most preferred range is around 1 µg/mL. The SFRP-2 protein may be a fusion protein engineered by fusing to a tag or a protein containing a mutation such as deletion in a portion whose function of is not affected to the protein, as long as the protein does not lose the function of improving hematopoietic ability and long-term bone marrow -repopulating ability in hematopoietic stem cells.

### == hematopoietic stem cell transplantation ==

Transplantation of hematopoietic stem cells grown as above may also be performed by a conventional method known to those skilled in the art. When transplanted hematopoietic stem cells have been integrated, their hematopoietic function in the transplanted recipient is recovered gradually. In this step, by improving the hematopoietic ability and long-term bone marrow-repopulating ability of hematopoietic stem cells, their ability of functional recovery is enhanced. When hematopoietic stem cells cultured in the presence of SFRP-2 protein according to the present invention are used for transplantation, the hematopoietic ability and long-term bone marrow-repopulating ability of the hematopoietic stem cells are improved, compared with hematopoietic stem cells cultured in the absence of SFRP-2 protein, as shown in the following examples. Therefore, SFRP-2 protein or expression vectors that can express SFRP-2 protein are useful as agents for improving the functions of hematopoietic stem cells. Moreover, a kit for culturing hematopoietic stem cells may be designed which includes SFRP-2 protein or an expression vector that can express SFRP-2 protein so that anyone can carry out easily the method according to the present invention. This kit may include a medium and hematopoietic stem cells, etc. in addition to SFRP-2 protein or an SFRP-2 protein-expressing expression vector.

Hematopoietic stem cell transplantation aims, for example, to recover the hematopoietic function in patients with diseases associated by decreased hematopoietic function, such as aplastic anemia. An alternative aim is to recover hematopoietic cells that have been damaged by pre-treatment with high-dose anticancer drug or radiation to kill tumor cells. In this alternative case, the therapy is applied to tumors susceptible to anti-cancer drugs and/or radiation: specifically, hematologic malignancies, such as leukemia, myelodysplastic syndromes, malignant lymphoma, and multiple myeloma, or certain type of solid cancer.

Hematopoietic stem cells to be used in this invention may be autologous hematopoietic stem cells or those obtained from a haploidentical related/unrelated donor who shares many HLA haplotypes with the recipient.

### EXAMPLES

### EXAMPLE 1

### In vitro maintenance of proliferation and multipotency of hematopoietic stem cells

CD34-KSL cell were isolated in the following procedure (Ema H et al., Nat. Protoc. 2006; vol. 1, p. 2979-2987). First, bone marrow cells were isolated from 8- to 12-week-old C57BL/6 mice (Clea Japan), and then monocytes were isolated from the bone marrow cells, using Lymphoprep (Nycomed Pharma AS). Next, the cell population was depleted of lineage-positive cells using Lineage Cell Depletion Kit (Miltenyi Biotec). The remaining cells were bound to FITC-conjugated anti-CD34 antibody, PE-conjugated anti-Sca-1 antibody, APC-conjugated anti-c-Kit antibody, and biotin-conjugated anti-lineage antibody cocktail in Lineage Cell Depletion Kit, followed by binding to streptavidin PE-Cy7. Then, using either FACS Calibur or FACS Aria, the cell populations were analyzed and CD34-KSL cells were isolated.

100 CD34-KSL cells were sorted at one cell per well into a 96-well culture plate, and cultured in S-clone SF03 (Sanko Junyaku) (containing 0.5% BSA, 50 ng/mL SCF, 50 ng/mL TPO, and 1 µg/mL SFRP-2) (cells obtained were defined as the SFRP-2 group) (denoted as SFRP-1 in the figures). Experiments were performed using a medium which did not contain SFRP-2 (R&D systems) as a negative control (cells obtained were defined as the control group) (denoted as Control in the figures) and, for the purpose of comparison, a medium containing SFRP-1 in place of SFRP-2(cells obtained were defined as the SFRP-1 group) (denoted as SFRP-1 in the figures).

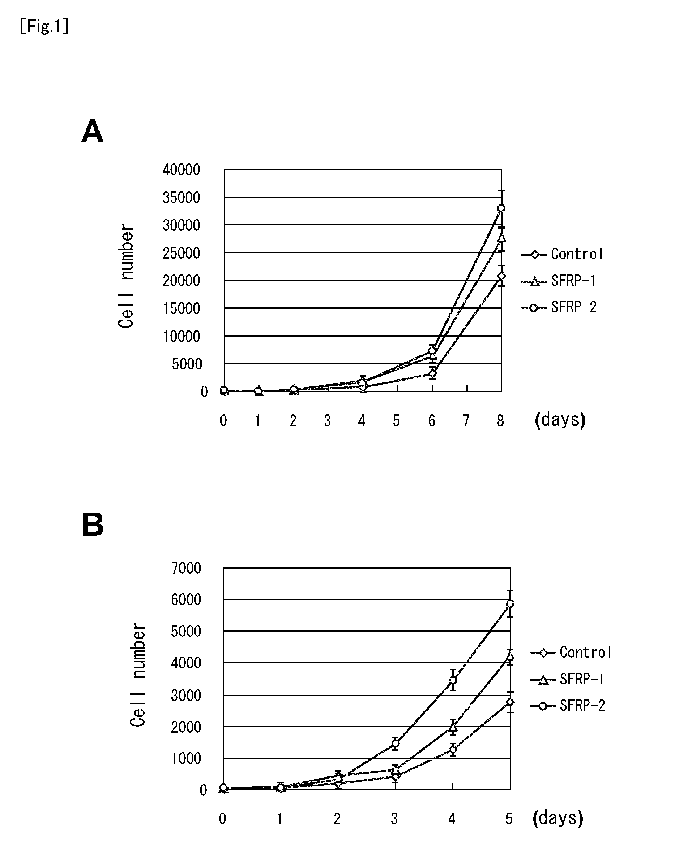

The cultured cells were observed and the number of the cells was measured under a microscope every 24 hours. As shown in FIG. 1A, both SFRP-1 and SFRP-2 promoted the proliferation of CD34-KSL cells, but SFRP-2 (about 1.5 fold) showed a higher proliferation-promoting ability than SFRP-1 (about 1.3 fold) .

Next, SFRP-1 or SFRP-2 expression vector was introduced into CD34-KSL cells and constitutively expressed in the cells. The effect of each protein on the proliferation ability of CD34-KSL cells was examined. First, by using as a template a cDNA derived from mouse bone marrow cells, the coding region of SFRP-1 or SFRP-2 was amplified by PCR with the following primers.
Primers used for SFRP-1:
   Forward 5'-ggatccatgggcgtcgggcgcagcgc-3' (SEQ ID NO: 1)
   Reverse 5'-gaattctcacttaaaaacagactgga-3' (SEQ ID NO: 2)
Primers used for SFRP-2
   Forward 5'-ggatccatgccgcggggccctgcctc-3' (SEQ ID NO: 3)
   Reverse 5'-gaattcctagcattgcagcttgcgga-3' (SEQ ID NO: 4)

The resulting DNA was digested with EcoRI and BamHI and was inserted into the EcoRI-BamHI sites of the expression vector pMXs-IG. The recombinant plasmid obtained was transfected into retrovirus-producing cells such as PLAT-E and BOSC23 cells. After culture for 48 h, the supernatant containing recombinant retrovirus was recovered. This supernatant was added to the medium of CD34-KSL cells (MOI= 30) to infect the recombinant retrovirus into the cells and SFRP-1 or SFRP-2 was forcibly expressed in CD34-KSL cells. Then, 100 recombinant CD34-KSL cells were seeded at one cell per well into a 96-well culture plate.

Cell proliferation was measured with the passage of time in the same manner as described above. Both SFRP-1 and SFRP-2 promoted the proliferation of CD34-KSL cells, but SFRP-2 (about 2-fold) showed a higher proliferation-promoting ability than SFRP-1 (about 1.5-fold).

Furthermore, colony assay was performed using CD34-KSL cells. Namely, isolated CD34-KSL cells were cultured in the same manner as described above. After 1 week (denoted as 1W in the figures) or 2 weeks (denoted as 2W in the figures), the medium was exchanged with a differentiation-inducing medium (S-clone SF03 (Sanko Junyaku) containing 10% FBS, 20 ng/ml SCF, 20 ng/ml IL-3, 50 ng/ml TPO, and 2-U/ml Epo). The cells were allowed to differentiate into each type by incubation for 7 more days, and recovered on glass slides for cytospin. Cell morphology was examined with May-Giemsa staining. The colonies formed were classified into the colonies containing erythrocytes and megakaryocytes (EM), colonies containing granulocytes and macrophages (GM), colonies containing granulocytes, erythrocytes, and macrophages (GEM), and colonies containing granulocytes, erythrocytes, macrophages, and megakaryocytes (GEMM) . The number of colonies for each was graphed.

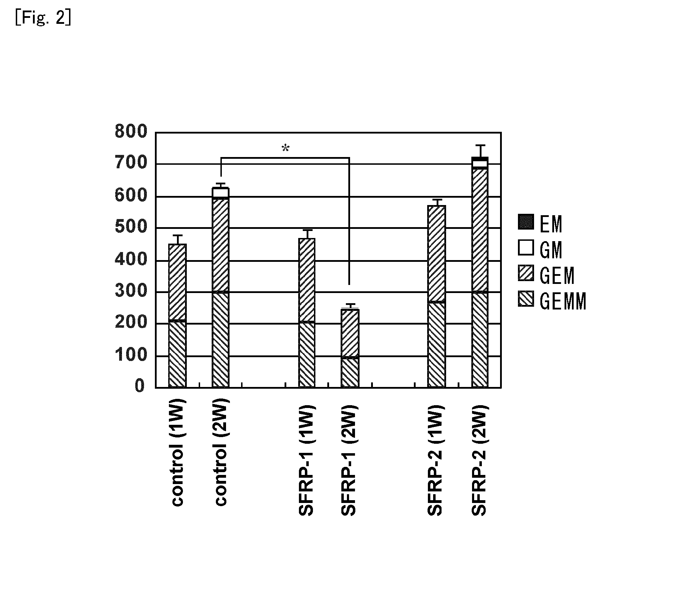

As a result shown in FIG. 2, in the SFRP-1 group, the number of multipotent progenitors (MPPs, including GEMs and GEMMs) was significantly decreased to about half of that in the control group. This indicates that differentiation of CD34-KSL cells was promoted during the 2-week culture in the presence of SFRP-1, and many of the cells lost their multipotency. In contrast, the number of MPPs in the SFRP-2 group was about 1.2-fold increased compared with that in the control group, showing that SFRP-2 maintains, or slightly enhances, the multipotency of CD34-KSL cells.

### EXAMPLE 2

### == Transplantation of CD34-KSL cells into mice and in vivo hematopoietic ability and long-term bone marrow-repopulating ability ==

Cells obtained by incubating 20 CD34-KSL cells isolated from B6-Ly5.1 mice (Sankyo-Lab Service) for 2 weeks in the same manner as in Example 1 were used as the donor cells. Meanwhile, B6-Ly5.2 mice were lethally irradiated at 950 rad, and the donor cells were transplanted into the mice together with 2 x 10⁵ monocytes isolated from the bone marrow of B6-Ly5.2 mice. The percentage of cells derived from the donor cells in peripheral blood was measured after 4 weeks, 8 weeks, 12 weeks, and 23 weeks (denoted as 4W, 8W, 12W, and 23W, respectively in the figures) . Since the cells derived from the donor cells were Ly5.1-positive, the percentage of the donor cell-derived cells was measured by measuring the percentage of Ly5.1-positive cells with FACS.

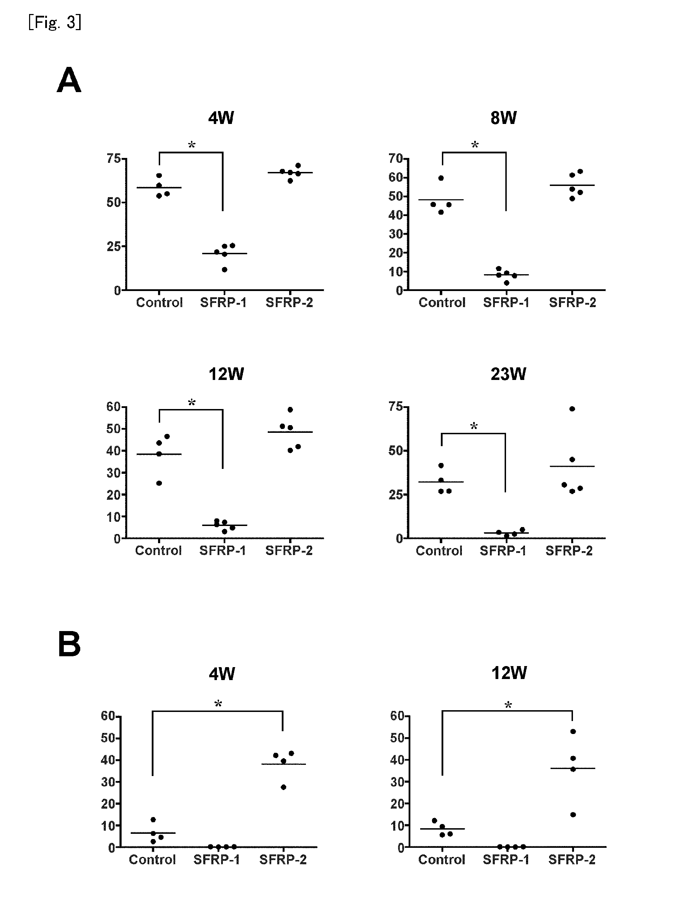

As shown in FIG. 3A, no significant difference was found between the control group and SFRP-2 group in the percentage at any measured time points. In contrast, when compared the control group and SFRP-1 group, the SFRP-1 group had a smaller number of CD34-KSL-derived cells at all measured time points.

Next, in order to compare the number of cells having long-term bone marrow-repopulating ability and hematopoietic ability in the bone marrow, bone marrow cells were harvested from mice 24 weeks after CD34-KSL cells were transplanted into the mice and transplanted secondarily into B6-Ly5.2 mice. In the secondary transplantation, 2 x 10⁶ bone marrow cells were transplanted into lethally irradiated (950 rad) B6-Ly5.2 mice. At 4 weeks and 12 weeks after the transplantation, the percentage of the donor cell-derived cells in peripheral blood was measured (denoted as 4W and 12W, respectively, in the figures).

As shown in FIG. 3B, in the primary transplantation, no significant difference was found in the percentage of the donor cells in peripheral blood between the control group and SFRP-2 group, whereas in the second transplantation, the percentage was significantly higher in the SFRP-2 group than that in the control group. This means that, in the primary transplantation, the percentage of hematopoietic stem cells which had integrated in the bone marrow was significantly higher in the SFRP-2 group than that in the control group. It should be noted that in SFRP-1 group, almost no cells were integrated in the bone marrow in the primary transplantation.

Thus, by culturing hematopoietic stem cells in the presence of SFRP-2 before transplantation into the living body, the integration rate in the bone marrow increases, and the hematopoietic ability and long-term bone marrow-repopulating ability are improved.

### INDUSTRIAL APPLICABILITY

The present invention has made it possible to provide a method for culturing hematopoietic stem cells, by which hematopoietic stem cells for hematopoietic stem cell transplantation can be produced.

## Claims

1. A method for culturing a hematopoietic stem cell, comprising the step of culturing the hematopoietic stem cell in the presence of a SFRP-2 protein.

2. The method of claim 1, comprising the step of adding the SFRP-2 protein to the medium for the hematopoietic stem cell.

3. The method of claim 1, comprising the step of introducing an expression vector of the SFRP-2 protein into the hematopoietic stem cell.

4. The method of any one of claims 1 to 3, wherein the concentration of the SFRP-2 protein in the medium is in the range of 0.1 µg/mL or more to 10 µg/mL or less.

5. The method of any one of claims 1 to 3, comprising the step of isolating a CD-34-negative, Sca-1-positive, c-Kit-positive, and lineage-antigen-negative hematopoietic stem cell from bone marrow cells or blood cells.

6. The method of any one of claims 1 to 3, wherein the lineage antigen is a set of antigens consisting of CD5, CD45R (B220), CD11b, Gr-1 (Ly-6G/C), 7-4, and Ter-119.

7. An agent for improving a function of a hematopoietic stem cell, comprising a SFRP-2 protein or an expression vector that expresses SFRP-2 protein.

8. A kit for culturing a hematopoietic stem cell, comprising a SFRP-2 protein or an expression vector that expresses the SFRP-2 protein.
